# EUROPEAN PATENT SPECIFICATION

(11) **EP 3 755 206 B1**
(45) Date of publication and mention of the grant of the patent: **07.06.2023**
(21) Application number: 19712857.2
(22) Date of filing: 25.02.2019
(51) Int. Cl.: A61B 3/15, G03B 17/56, A61B 5/00

(54) **PORTABLE MODULAR SYSTEM FOR DIAGNOSTIC INSTRUMENTS**
TRAGBARES MODULARES SYSTEM FÜR DIAGNOSTISCHE INSTRUMENTE
SYSTÈME MODULAIRE PORTABLE POUR INSTRUMENTS DE DIAGNOSTIC

(30) Priority: 23.02.2018 IT 201800003013
(43) Date of publication of application: 30.12.2020
(73) Proprietor: Espansione Marketing S.p.A., 40050 Funo (BO) (IT)
(72) Inventor: POMAR, Rodolfo, 40050 Bologna (IT)
(74) Representative: Manzella & Associati
(86) International application number: PCT/IT2019/050040
(87) International publication number: WO 2019/162981

(56) References cited:
- WO-A1-2015/049404
- US-A1- 2010 097 573
- US-A1- 2012 320 340
- Shoulderpod: "SHOULDERPOD GOES FULLY MODULAR", , 27 June 2017 (2017-06-27), XP055599636, Retrieved from the Internet: URL:https://www.shoulderpod.com/s/Shoulder pod_Press_release_June_2017.pdf [retrieved on 2019-06-26]

## Description

### Technical field

The present invention relates to a portable modular system for medical instruments, in particular for diagnostic instruments.

### Prior art

The use of specific medical instruments is known to perform diagnostic procedures as well as therapeutic treatment.

In particular, apparatuses are known which are capable of diagnosing pathologies involving, for example, the eyelids, causing chronic inflammatory states of the periorbital region. Such pathologies occur for example with an alteration of the lacrimal production, which may be excessive, insufficient or however qualitatively altered, as in the case of dry eye syndrome. The known pathologies that can manifest these symptoms are, for example, blepharitis, chalazion, stye and meibomitis.

To make a diagnosis of these dysfunctions, diagnostic instruments commonly used in specialized medical facilities or hospitals are known. These instruments are able to detect images of the interested parts, to enlarge them in an appropriate way, so that they can be analyzed by a specialist.

It is by now well-acknowledged fact that with an early diagnosis of these pathologies, as indeed in general, it is possible to considerably increase the possibility of recovery, the effectiveness as well as the speed of action of the available therapeutic methods.

For example, in the field of dermatology and ophthalmic medicine, the aforementioned specialized medical facilities have very complex diagnostic instruments, thus expensive, equipped with a camera for cupturing images of the parts of the body under examination, which allow the specialists to thoroughly value the symptomatic status of the patient, so as to make safely an early diagnosis.

Therefore, it often happens that only during specialist checks, it is possible to provide a diagnosis of the disease, thus increasing the risk of a late diagnosis, when the disease, in an advanced state of development, shows clear symptoms.

US 2012/320340 discloses a portable diagnostic instrument comprising: a viewing instrument or an analog or digital camera, an image capture device such as a smartphone, a bracket for supporting the smartphone and a locking ring for connection to a handle.

### Disclosure

The object of the present invention is to solve the mentioned problems, devising a portable modular system for diagnostic instruments which enables the diagnostic procedures to to be carried out easily.

Within this task, it is a further object of the present invention to provide a portable modular system for diagnostic instruments that is easily transportable, as well as usable for performing diagnostic procedures even outside specialised facilities or hospitals.

A further object of the invention is to provide a portable modular system for diagnostic instruments which is simple in construction and functional design, has certainly reliable operation, versatile use, as well as a relatively inexpensive cost.

The aforementioned objects are achieved, according to the present invention, by the portable modular system according to claim 1.

The modular system for diagnostic instruments comprises a first operating module, a second operating module, a support member intended to support in a removable manner and in respective positions the aforementioned first operating module and second operating module, as well as a coupling member connected to the support member to enable interchangeable coupling to a base body or to a handle for manual support of the modular system itself.

The aforementioned support member comprises at least a first seat for fixing the first operating module, said seat being intended to perform a guiding function for fixing.

The aforementioned support member comprises at least a second seat for fixing the second operating module, this seat being intended to perform a guiding function for fixing.

The aforementioned first operating module comprises or consists of a data detection and acquisition device, in particular of images, for example a video camera or a camera, so as to allow the efficient execution of diagnostic procedures by means of the modular system.

Advantageously, the second operating module comprises or consists of an electronic data processing unit, configured to receive and process the images acquired by the detection and acquisition device.

According to a particular aspect of the invention, in a very useful way, the aforementioned processing unit is integrated in a tablet, therefore it is very easy to transport and to use it.

The detection device can be made by means of an optical head on which different types of optics and cameras can be installed interchangeably, with various spectra, for example with infrared optics and/or cameras, white light and/or blue light.

The support element of the modular system is preferably made by means of a bracket having said at least one first seat, carrying said first operating module, and said second seat, carrying said second operating module, preferably in the form of guide slots. This structure is very easy to assemble and disassemble.

The aforementioned bracket is preferably L-shaped, comprising a first arm, a second arm and an intermediate portion. This structure is particularly advantageous for correctly orienting a detection device towards the patient, as well as the processing unit, if provided, with respect to the operator.

Advantageously, the aforementioned first operating module is applied to the support member at the aforementioned first seat by means of adjustable fixing means, for example with the aid of manual adjustment knobs.

Advantageously, the image detection and acquisition device comprises or consists of an infrared video camera or an infrared camera, for diagnosing pathologies affecting the eyelids.

Advantageously, the first seat is slot shaped.

Advantageously, the second seat is slot shaped.

Advantageously, the first seat is made at the first arm and the second seat is made at the intermediate portion of the bracket.

Advantageously, the intermediate portion, comprising the second slot-shaped seat, has an arched shape, configured so as to allow the angle of inclination of the second operating module to be adjusted with respect to the first operating module by adjusting the position of the second operating module in the second seat.

The aforementioned coupling member preferably comprises connectable coupling means for fixing to the aforementioned base body or to the aforementioned handle.

### Description of drawings

The details of the invention will become more evident from the detailed description of a preferred embodiment of the portable modular system for diagnostic instruments, according to the invention, illustrated by way of example in the accompanying drawings, in which:
Figure 1 shows a perspective view of the modular system according to the invention;
Figure 2 shows a perspective view of the same system according to a different usage.

### Embodiments of the invention

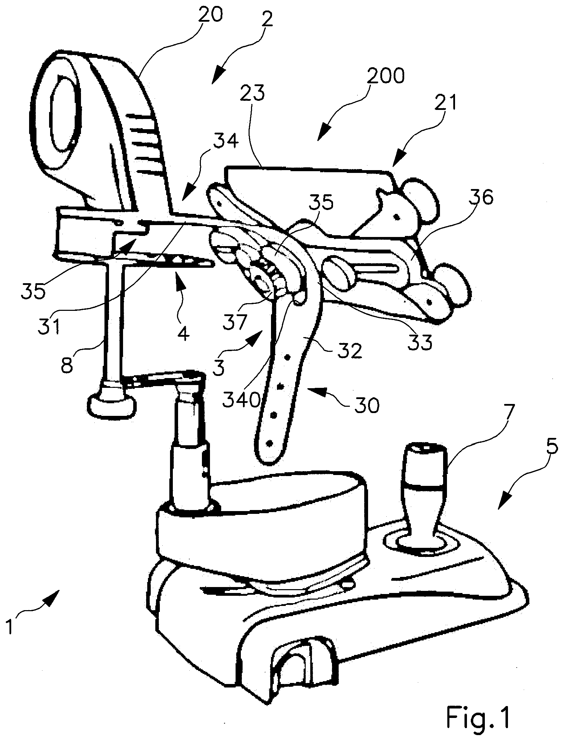

With particular reference to these figures, the reference numeral 1 generally designates the portable modular system for diagnostic instruments (see Figure 1).

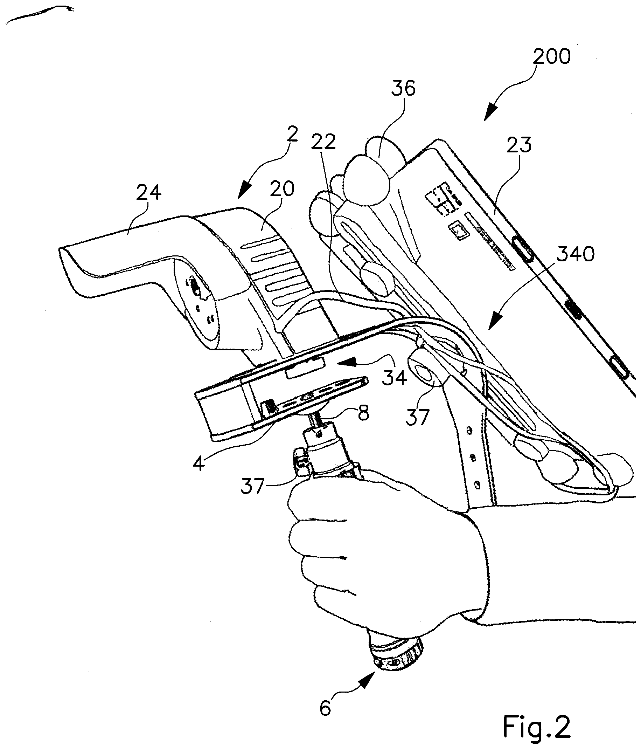

The modular system 1 comprises a first operating module 2, a second operating module 200, a support member 3 intended to support the aforementioned first operating module 2 and a second operating module 200, as well as a coupling member 4 connected to the support member 3 in order to enable coupling to a base body 5 or to a handle 6 (see Figures 1 and 2) for manual support of the modular system 1 itself, as described in detail below.

The first operating module 2, applicable to the support member 3, includes at least one data detection and acquisition device 20, in particular images, in particular a video camera or a camera.

The second operating module 200 advantageously comprises an electronic data processing unit 21, configured to receive and process the images acquired by the detection and acquisition device 20.

The detection and acquisition device 20 can be made for example by means of an optical head on which different types of optics and cameras can be installed interchangeably, with different spectra, in particular with infrared optics and/or cameras, white light and/or blue light.

According to a preferred embodiment, the detection and acquisition device 20 comprises an infrared video camera or an infrared camera.

The detection and acquisition device 20 and the processing unit 21 are advantageously connected, for example by cables or alternatively wireless, so that the data, particularly images, detected by the first, can be effectively processed, in real time, by the second, for example to extract, record, file, send, processed diagnostic data.

The processing unit 21 can be advantageously integrated into a portable tablet 23, therefore it is easily transportable and usable.

The support member 3 can be made up of a bracket 30, for example L-shaped, thus comprising a first arm 31, a second arm 32 and an intermediate connecting portion 33.

The bracket 30 comprises at least one first seat 34 for the application of the first operating module 2, adapted to perform a guiding function for fixing.

More precisely, the provided seat 34 enables the first operating module 2 to be applied to the support member 3 in a stable and removable manner. This application takes place by means of fixing means 35 of a known type, for example of the clamp type or the like (see Figure 1).

The bracket 30 also comprises a second seat 340 for the application of the second operating module 200.

In the illustrated case, for example, the support member 3 comprises a first seat 34 at the mentioned first arm 31, for the application of the detection device 20 and a second seat 340 at the intermediate connecting portion 33, for the positioning of the tablet 23 which integrates the processing unit 21. In particular, the tablet 23 is applied by means of a support rod 36 engaged to the respective seat 340 by screw-on fixing means 35.

Advantageously, the fixing means 35 comprises an adjustment knob 37, adapted to ease the adjustment of the fixing, adapting the positioning, thus the orientation of the first operating module and of the second operating module to the specific requirements.

In particular, it is therefore possible to adjust the orientation of both the detection device 20 and the tablet 23 which advantageously integrates the processing unit 21 acting independently on each adjustment knob 37.

In any case, it is possible to apply the first operating module 2 and the second operating module 200 firmly to the support member 3, so that, for example, the detection member 20 faces the patient, at a zone of the body to be observed, and the processing unit 21, instead, faces the operator.

In the illustrated embodiment, the first seat 34 and the second seat 340 are slot shaped.

In particular, the intermediate portion 33, on which the second slot-shaped seat 340 is formed, has an arched shape, configured so as to allow the adjustment of the angle of inclination of the second operating module 200 with respect to the first operating module 2.

The same guide slots 34, 340 or different slots, formed on the same support member 3, can act as useful passages for the cables 22, if provided.

As anticipated, the modular system 1 can be easily fixed on a base body 5 or to a special handle 6 by means of the coupling member 4 fixed to the support member 3.

The coupling member 4 may for example be provided near a seat 34 for the application of the first operating module 2, so as to control its orientation more directly, by orientation means 7 possibly provided by the base body 5 or manually by the handle 6. In the shown case for example (see Figures 1 and 2), the coupling member 4 is fixedly positioned at the end of the first arm 31 of the support member 3.

The coupling member 4 can be interchangeably fixed to the base body 5 of a medical device, for example a slit lamp for ophthalmic investigations. The application can be carried out simply by interposing an adjustable coupling member 8 by acting on a respective knob 37.

Alternatively, the coupling member 4 can be connected, in the same way or with equivalent means, to a support stand.

As described above, the coupling member 4 can preferably be engaged directly to a handle 6, so as to allow the operator to support the modular system 1, thus operating the correct positioning of the supported medical equipment with respect to the patient.

Moreover, the detection device 20 can be associated with a spacer element 24, adapted to ease the correct positioning with respect to the patient, interposed between the patient and the device itself, specially in the case of manual support through the handle 6 (see in particular Figure 2).

The operation of the modular system according to the invention is easily understandable from the above description.

In an initial phase the modular system is preferably arranged inside a transportable container, for example a briefcase, in which the various components are stored in a disassembled condition.

If necessary, the operator assembles the first operating module 2 and the second operating module 200 on the support member 3, adjusting them at the seats 34, 340 by the fixing means 35 and the knobs 37.

In particular, the operator provides to orient the detection device 20 towards the patient and towards himself the processing unit 21. In the same preparation step the operator can fix the support member 3 on the base body 5 of an installed apparatus, for example at an ophthalmic ambulatory, or at handle 6, in this case for manual use for example at a paramedical center or a pharmacy.

The operator can then activate the detection device 20 and the processing unit 21, to start the diagnostic procedure.

## Claims

1. Portable modular system for diagnostic instruments in the field of dermatological, ophthalmic and optometry medicine, the system comprising a first operating module (2), comprising at least one image detection and acquisition device (20), in particular a video camera or a camera; a second operating module (200), comprising an electronic data processing unit (21), configured to receive and process the images obtained by said image detection and acquisition device (20); a support member (3) intended to support in a removable manner and in respective positions said first operating module (2) and said second operating module (200); a coupling member (4) connected to said support member (3) in order to enable interchangeable coupling to a base body (5) or to a handle (6) for manual support of said modular system (1), said support member (3) comprising at least a first seat (34) for fixing said first operating module (2) and a second seat (340) for fixing said second operating module (200), said first seat (34) and second seat (340) being predisposed to act as a guide for said fixing.

2. Modular system according to claim 1, **characterized in that** said image detection and acquisition device (20) comprises an infrared video camera or an infrared camera.

3. Modular system according to claim 1, **characterized in that** said first operating module (2) is made by means of an optical head on which different types of optics and cameras can be installed interchangeably, with various spectra, in particular with infrared optics and/or cameras, white light and/or blue light.

4. Modular system according to claim 1, **characterized in that** said processing unit (21) is integrated in a tablet (23).

5. Modular system according to one of claims 1 to 4, **characterized in that** said support member (3) is made by means of a bracket (30) carrying said first seat (34) and said second seat (340).

6. Modular system according to claim 5, **characterized in that** said first operating module (2) and said second operating module (200) are applied to said support member (3) respectively at said first seat (34) and at said second seat (340) by means of adjustable fixing means (35).

7. Modular system according to claim 6, **characterized in that** said fixing means (35) comprise respective knobs (37) for the manual adjustment of the positioning of said first module (2) and second module (200).

8. Modular system according to one of the preceding claims, **characterized in that** said coupling member (4) comprises connectable coupling means for fixing to said base body (5) or to said handle (6).

9. Modular system according to one of claims 1 to 8, **characterized in that** said first seat (34) and second seat (340) are slot shaped.

10. Modular system according to one of claims 5 to 9, **characterized in that** said bracket (30) is L-shaped, comprising a first arm (31), a second arm (32) and an intermediate connecting portion (33).

11. Modular system according to claim 10, **characterized in that** said first seat (34) is made at said first arm (31) and said second seat (340) is made at said intermediate portion (33).

12. Modular system according to claim 11, **characterized in that** said intermediate portion (33), comprising said second slot shaped seat (340) has an arched shape, configured so as to enable adjusting the angle of inclination of said second operating module (200) with respect to said first operating module (2) by adjusting the position of said second operating module (200) in said second seat (340).

13. Modular system according to one of the preceding claims, **characterized in that** said at least one image detection and acquisition device (20) can be associated with a spacer element (24), adapted to facilitate correct positioning with respect to the patient, interposed between the patient and the device itself.

14. Modular system according to one of the preceding claims, **characterized in that** said positions in which said support member (3) is intended to support said first operating module (2) and said second operating module (200) are adjustable.

## Patentansprüche

1. Tragbares modulares System für diagnostische Instrumente im Bereich der dermatologischen, ophthalmischen und optometrischen Medizin, wobei das System ein erstes Bedienmodul (2) umfasst, das mindestens eine Bilderfassungs- und -aufzeichnungsvorrichtung (20), insbesondere eine Videokamera oder eine Kamera, umfasst; ein zweites Bedienmodul (200), das eine elektronische Datenverarbeitungseinheit (21) umfasst, die konfiguriert ist, um die Bilder, die von der Bilderfassungs- und -aufzeichungsvorrichtung (20) erhalten werden, zu empfangen und zu verarbeiten; ein Trägerelement (3), das dazu bestimmt ist, das erste Bedienmodul (2) und das zweite Bedienmodul (200) in einer abnehmbaren Weise und in jeweiligen Positionen zu tragen; ein Kopplungselement (4), das mit dem Trägerelement (3) verbunden ist, um ein austauschbares Koppeln an einem Basiskörper (5) oder an einem Griff (6) zum manuellen Tragen des modularen Systems (1) zu ermöglichen, wobei das Trägerelement (3) mindestens einen ersten Sitz (34) zum Befestigen des ersten Bedienmoduls (2) und einen zweiten Sitz (340) zum Befestigen des zweiten Bedienmoduls (200) umfasst, wobei der erste Sitz (34) und der zweite Sitz (340) dazu prädisponiert sind, als eine Führung für das Befestigen zu wirken.

2. Modulares System nach Anspruch 1, **dadurch gekennzeichnet, dass** die Bilderfassungs- und -aufzeichnungsvorrichtung (20) eine Infrarotvideokamera oder eine Infrarotkamera umfasst.

3. Modulares System nach Anspruch 1, **dadurch gekennzeichnet, dass** das erste Bedienmodul (2) mittels eines optischen Kopfes hergestellt ist, auf dem unterschiedliche Arten von Optiken und Kameras austauschbar mit verschiedenen Spektren, insbesondere mit Infrarotoptiken und/oder Kameras, weißem Licht und/oder blauem Licht, installiert werden können.

4. Modulares System nach Anspruch 1, **dadurch gekennzeichnet, dass** die Verarbeitungseinheit (21) in einem Tablet (23) integriert ist.

5. Modulares System nach einem der Ansprüche 1 bis 4, **dadurch gekennzeichnet, dass** das Trägerelement (3) mittels einer Halterung (30) hergestellt ist, die den ersten Sitz (34) und den zweiten Sitz (340) trägt.

6. Modulares System nach Anspruch 5, **dadurch gekennzeichnet, dass** das erste Bedienmodul (2) und das zweite Bedienmodul (200) an dem Trägerelement (3) jeweils an dem ersten Sitz (34) und an dem zweiten Sitz (340) mittels einstellbarer Befestigungsmittel (35) angebracht sind.

7. Modulares System nach Anspruch 6, **dadurch gekennzeichnet, dass** die Befestigungsmittel (35) jeweilige Knöpfe (37) für die manuelle Einstellung der Positionierung des ersten Moduls (2) und des zweiten Moduls (200) umfassen.

8. Modulares System nach einem der vorstehenden Ansprüche, **dadurch gekennzeichnet, dass** das Kopplungselement (4) verbindbare Kopplungsmittel zum Befestigen an dem Basiskörper (5) oder an dem Griff (6) umfasst.

9. Modulares System nach einem der Ansprüche 1 bis 8, **dadurch gekennzeichnet, dass** der erste Sitz (34) und der zweite Sitz (340) schlitzförmig sind.

10. Modulares System nach einem der Ansprüche 5 bis 9, **dadurch gekennzeichnet, dass** die Halterung (30) L-förmig ist, einen ersten Arm (31), einen zweiten Arm (32) und einen Zwischenverbindungsabschnitt (33) umfasst.

11. Modulares System nach Anspruch 10, **dadurch gekennzeichnet, dass** der erste Sitz (34) an dem ersten Arm (31) gebildet ist und der zweite Sitz (340) an dem Zwischenabschnitt (33) gebildet ist.

12. Modulares System nach Anspruch 11, **dadurch gekennzeichnet, dass** der Zwischenabschnitt (33), der den zweiten schlitzförmigen Sitz (340) umfasst, eine bogenförmige Form aufweist, die konfiguriert ist, um das Einstellen des Neigungswinkels des zweiten Bedienmoduls (200) in Bezug auf das erste Bedienmodul (2) durch Einstellen der Position des zweiten Bedienmoduls (200) in dem zweiten Sitz (340) zu ermöglichen.

13. Modulares System nach einem der vorstehenden Ansprüche, **dadurch gekennzeichnet, dass** die mindestens eine Bilderfassungs- und - aufzeichnungsvorrichtung (20) mit einem Abstandselement (24) assoziiert sein kann, das angepasst ist, um eine korrekte Positionierung in Bezug auf den Patienten zu erleichtern, und das zwischen dem Patienten und der Vorrichtung selbst eingefügt ist.

14. Modulares System nach einem der vorstehenden Ansprüche, **dadurch gekennzeichnet, dass** die Positionen, in denen das Trägerelement (3) das erste Bedienmodul (2) und das zweite Bedienmodul (200) tragen soll, einstellbar sind.

## Revendications

1. Système modulaire portable pour instruments de diagnostic dans le domaine de la médecine dermatologique, ophtalmique et optométrique, le système comprenant un premier module de fonctionnement (2), comprenant au moins un dispositif de détection et d'acquisition d'images (20), en particulier une caméra vidéo ou une caméra; un second module de fonctionnement (200), comprenant une unité de traitement de données électroniques (21), configurée pour recevoir et traiter les images obtenues par ledit dispositif de détection et d'acquisition d'images (20); un élément de support (3) destiné à supporter de manière amovible et dans des positions respectives ledit premier module de fonctionnement (2) et ledit second module de fonctionnement (200); un élément de couplage (4) relié audit élément de support (3) afin de permettre un couplage interchangeable à un corps de base (5) ou à une poignée (6) pour un support manuel dudit système modulaire (1), ledit élément de support (3) comprenant au moins un premier siège (34) pour fixer ledit premier module de fonctionnement (2) et un second siège (340) pour fixer ledit second module de fonctionnement (200), lesdits premier siège (34) et second siège (340) étant prédisposés à agir comme guide pour ladite fixation.

2. Système modulaire selon la revendication 1, **caractérisé en ce que** ledit dispositif de détection et d'acquisition d'images (20) comprend une caméra vidéo infrarouge ou une caméra infrarouge.

3. Système modulaire selon la revendication 1, **caractérisé en ce que** ledit premier module de fonctionnement (2) est réalisé au moyen d'une tête optique sur laquelle différents types d'optiques et de caméras peuvent être installés de manière interchangeable, avec différents spectres, en particulier avec des optiques et/ou des caméras infrarouges, une lumière blanche et/ou une lumière bleue.

4. Système modulaire selon la revendication 1, **caractérisé en ce que** ladite unité de traitement (21) est intégrée dans une tablette (23).

5. Système modulaire selon l'une des revendications 1 à 4, **caractérisé en ce que** ledit élément de support (3) est réalisé au moyen d'un support (30) portant ledit premier siège (34) et ledit second siège (340).

6. Système modulaire selon la revendication 5, **caractérisé en ce que** ledit premier module de fonctionnement (2) et ledit second module de fonctionnement (200) sont appliqués audit élément de support (3) respectivement au niveau dudit premier siège (34) et au niveau dudit second siège (340) au moyen de moyens de fixation réglables (35).

7. Système modulaire selon la revendication 6, **caractérisé en ce que** lesdits moyens de fixation (35) comprennent des boutons respectifs (37) pour le réglage manuel du positionnement dudit premier module (2) et second module (200).

8. Système modulaire selon l'une des revendications précédentes, **caractérisé en ce que** ledit élément de couplage (4) comprend des moyens de couplage connectables pour la fixation audit corps de base (5) ou à ladite poignée (6).

9. Système modulaire selon l'une des revendications 1 à 8, **caractérisé en ce que** ledit premier siège (34) et ledit second siège (340) sont en forme de fente.

10. Système modulaire selon l'une des revendications 5 à 9, **caractérisé en ce que** ledit support (30) est en forme de L, comprenant un premier bras (31), un second bras (32) et une partie de connexion intermédiaire (33).

11. Système modulaire selon la revendication 10, **caractérisé en ce que** ledit premier siège (34) est réalisé au niveau dudit premier bras (31) et ledit second siège (340) est réalisé au niveau de ladite partie intermédiaire (33).

12. Système modulaire selon la revendication 11, **caractérisé en ce que** ladite partie intermédiaire (33), comprenant ledit second siège en forme de fente (340) a une forme arquée, configurée de manière à permettre le réglage de l'angle d'inclinaison dudit second module de fonctionnement (200) par rapport audit premier module de fonctionnement (2) en ajustant la position dudit second module de fonctionnement (200) dans ledit second siège (340).

13. Système modulaire selon l'une des revendications précédentes, **caractérisé en ce que** ledit au moins un dispositif de détection et d'acquisition d'images (20) peut être associé à un élément d'espacement (24), adapté pour faciliter un positionnement correct par rapport au patient, interposé entre le patient et le dispositif lui-même.

14. Système modulaire selon l'une des revendications précédentes, **caractérisé en ce que** lesdites positions dans lesquelles ledit élément de support (3) est destiné à supporter ledit premier module de fonctionnement (2) et ledit second module de fonctionnement (200) sont réglables.
